(19) Europäisches Patentamt
European Patent Office
Office européen des brevets

(11) EP 0 512 754 B1

(12) **EUROPEAN PATENT SPECIFICATION**

(45) Date of publication and mention of the grant of the patent:
**05.07.2000 Bulletin 2000/27**

(51) Int. Cl.$^7$: **C07D 498/18**, A61K 31/445 // (C07D498/18, 311:00, 273:00, 221:00)

(21) Application number: **92303916.8**

(22) Date of filing: **30.04.1992**

(54) **Reduction products of rapamycin as immunosuppressants antiinflammatory or antifungal agents**

Reduktionsprodukte von Rapamycin als immunosuppressive, entzündungshemmende und antifungale Mittel

Produits de réduction de rapamycine comme agents immunosuppresants, anti-inflammatoires et anti-fongiques

(84) Designated Contracting States:
**AT BE CH DE DK ES FR GB GR IT LI LU NL PT SE**

(30) Priority: **07.05.1991 US 696692**
**07.08.1991 US 741714**

(43) Date of publication of application:
**11.11.1992 Bulletin 1992/46**

(73) Proprietor:
**AMERICAN HOME PRODUCTS CORPORATION**
**Madison, New Jersey 07940-0874 (US)**

(72) Inventors:
- **Caufield, Craig Eugene**
  **Plainsboro, Middlesex, New Jersey (US)**
- **Hughes, Philip Floyd**
  **Chapel Hill, North Carolina 27514 (US)**
- **Musser, John Henry**
  **Alameda, California 94501 (US)**

(74) Representative:
**Wileman, David Francis, Dr. et al**
**c/o Wyeth Laboratories**
**Huntercombe Lane South**
**Taplow Maidenhead Berkshire SL6 OPH (GB)**

(56) References cited:
**US-A- 5 023 262**

- **CANADIAN JOURNAL OF CHEMISTRY. vol. 60, no. 15, 1982, OTTAWA CA pages 2046 - 2047; J. A. FINDLAY ET AL.: 'The structure of demethoxyrapamycin'**

EP 0 512 754 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to reduction products derived from Rapamycin where the ketone functionality at positions 15, 33, 15 and 27, 15 and 33 or 15, 27 and 33 is replaced with a hydroxy group, which are useful in the treatment of transplantation rejection, autoimmune diseases (i.e lupus, rheumatoid arthritis, diabetes mellitus, multiple sclerosis), Candida albicans infections, and diseases of inflammation; to their preparation and to pharmaceutical compositions containing them.

Rapamycin is a macrocyclic triene antibiotic produced by Streptomyces hygroscopicus, which was found to have antifungal activity, particularly against Candida albicans both in vitro and in vivo [C. Vezina et al., J. Antibiot. 28, 721 (1975); S.N. Seghal et al., J. Antibiot. 28, 727 (1975); H. A. Baker et al., J. Antibiot. 31, 539 (1978); U.S. Patent 3,929,992; and U.S. Patent 3,993,749].

Rapamycin alone (U.S. Patent 4,885,171) or in combination with picibanil (U.S. Patent 4,401,653) has been shown to have antitumor activity. R. Martel et al. [Can. J. Physiol. Pharmacol. 55, 48 (1977)] disclosed that rapamycin is effective in the experimental allergic encephalomyelitis model, a model for multiple sclerosis; in the adjuvant arthritis model, a model for rheumatoid arthritis; and effectively inhibited the formation of IgE-like antibodies.

The immunosuppressive effects of rapamycin have been disclosed in FASEB 3, 3411 ( 1989). Rapamycin has been shown to be effective in inhibiting transplant rejection (U.S. Patent Application Ser. No. 362,544 filed June 6, 1989). Cyclosporin A and FK-506, other macrocyclic molecules, also have been shown to be effective as immunosuppressive agents, therefore useful in preventing transplant rejection [FASEB 3, 3411 (1989); FASEB 3, 5256 (1989); and R. Y. Calne et al., Lancet 1183 (1978)].

Mono- and diacylated derivatives of rapamycin (esterified at the 28 and 43 positions) have been shown to be useful as antifungal agents (U.S. Patent 4,316,885) and used to make water soluble prodrugs of rapamycin (U.S. Patent 4,650,803). Recently, the numbering convention for rapamycin has been changed; therefore according to Chemical Abstracts nomenclature, the esters described above would be at the 31- and 42- positions.

This invention provides derivatives of rapamycin which are useful as immunosuppressive, antiinflammatory, antifungal and antitumor agents. The invention compounds thus are useful in the treatment of transplant rejection, autoimmune diseases, Candida albicans infections, and diseases of inflammation.

The compounds of this invention are represented by Formula I below wherein the groups X, Y, and Z at positions 15, 27 and 33 respectively are -CO- or -CHOH-and at least one of X or Z must be -CHOH and when Y is CHOH then X is CHOH:

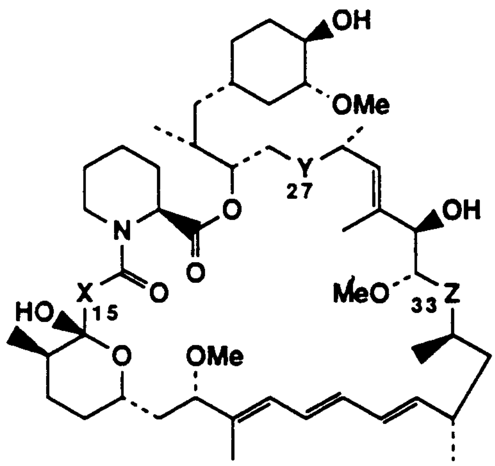

Formula I

Formula I also encompasses the pharmaceutically acceptable salts which may be formed from inorganic cations such as sodium, potassium and the like.

This invention also provides processes for preparing the compounds of formula I. Accordingly this invention provides a process for preparing a compound of formula I which comprises reducing rapamycin with one or sequentially in either order with two reducing agents, said reducing agents being selected from diisobutylaluminium hydride (or similar standard ketone reagent such as a boron or aluminium reagent of formula $R_2MH$ where R is a ligand and M is Al or B) and a β-hydroxyketone reducing agent such as sodium triacetoxyborohydride or other similar boron or aluminium 'ate' reagent of formula $R_3MH^-M'^+$ where $M'^+$ is an alkali metal.

In detail the formula I compounds are prepared by reduction of rapamycin with a suitable reducing agent. Diisobutylaluminium hydride (DIBAL) or similar reagent will reduce the ketone functionalities of rapamycin at positions 15 or 15 and 27, depending on the reaction conditions. The ketone functionality at position 33 of rapamycin is reduced selectively with sodium tracetoxyborohydride, a reagent reported to selectively reduce β-hydroxyketones predominantly in an anti-fashion (Evans, D.A.; Chapman, K. T.; Carreira, E. M.; J Amer. Chem. Soc. 1988, 110, 3560-3568) or by using other reagents known to reduce β-hydroxyketones. If the hydroxy groups at positions 31 and 42 are protected by a hydroxy protecting agent e.g as the t-butyldimethylsilyl ethers then reduction using an 'ate' reagent of formula $R_3MH^-M'^+$ e.g potassium triphenyl borohydride occurs at position 15. (Silyl ethers of rapamycin are disclosed in EPA 92302843.5 filed 31 March 1992) If the hydroxy groups are not protected then reduction at position 15 may follow the reduction at position 33. Thus reduction of rapamycin with sodium triacetoxyborohydride in tetrahydrofuran (THF) yields the 33-hydroxy analog or 33-deoxo-33-hydroxyrapamycin shown as Compound II in Scheme A. The predominant R isomer was obtained upon chromatographic separation of the reaction products. Further reduction gives the 15,33-dihydroxy analog.

Scheme A: Reduction of Rapamycin at Position 33.

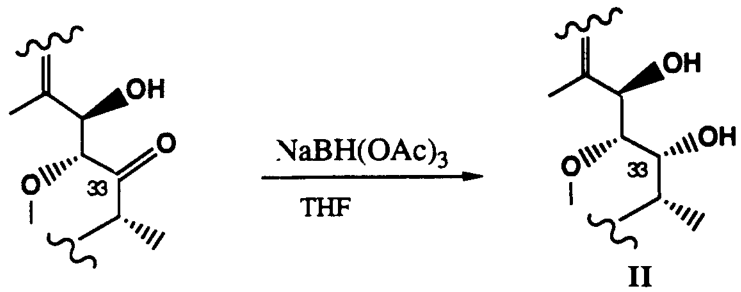


II

Rapamycin

33-deoxo-33-(R)-hydroxy-rapamycin

Rapamycin can be cleaved between the C-31 and C-32 carbons due to base degradation attributed to a reverse aldol reaction. Reduction of the C-33 ketone functionality removes this pathway for decomposition.

Reduction of rapamycin with DIBAL at -78°C in THF results in the C-15 ketone group being reduced as shown in the following reaction scheme.

Scheme B: Reduction of Rapamycin at Position 15.

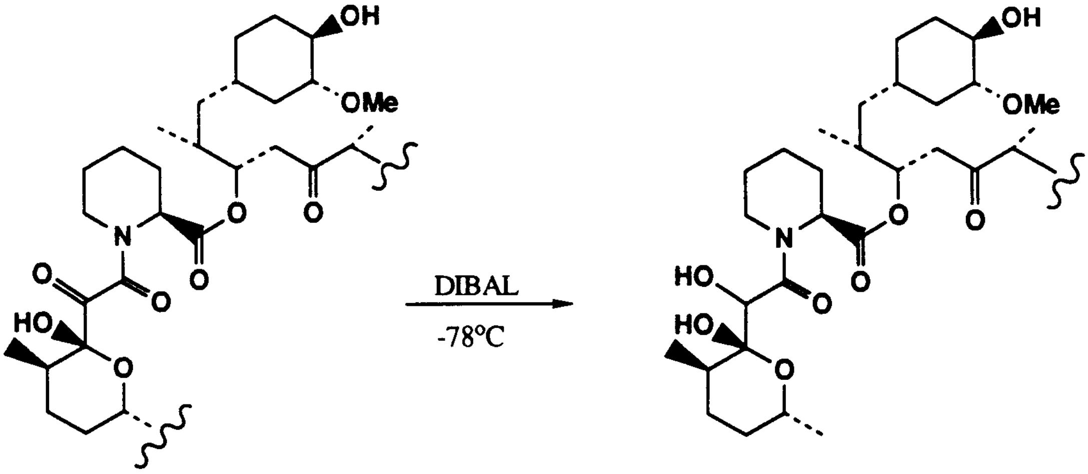


When the reaction mixture is allowed to proceed at -20°C following addition of DIBAL at -78°C, reduction of the keto groups at positions 15 and 27 occurs giving the 15, 27-diol as shown in Scheme C.

Scheme C. Reduction of Rapamycin at positions 15 and 27

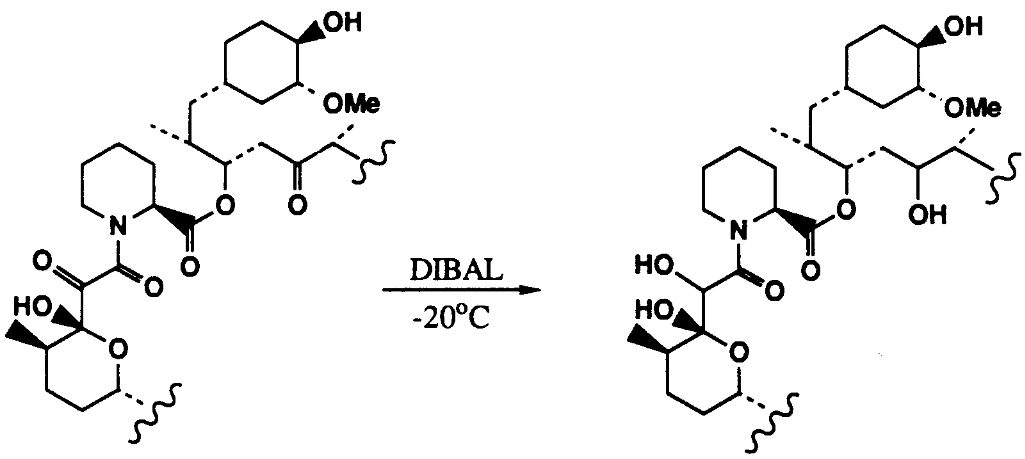


Diastereomers obtained in Scheme C are separated by chromatographic procedures, i.e., preparative high pressure liquid chromatography.

Reduction of the two carbonyl groups X and Z and three carbonyl groups X, Y and Z of rapamycin can be accomplished by sequential reduction steps. Still other reducing agents known to those skilled in the art may be used to effect selective reductions at these positions - see for example the publication by Evans et al, cited above.

The following examples illustrate the preparation of the compounds of this invention.

Example 1.

15-Deoxo-15-hydroxyrapamycin

To a solution of 750 mg (821 µmol) of rapamycin in 15 mL of dry THF was added dropwise at -78°C, 3.6 mL (3.6 mmol) of a 1.0 M solution of DIBAL in hexanes. After 1 h, the reaction was worked up by adding 1.0 N HCl to dissolve the aluminum salts. The aqueous solution was extracted three times with ethyl acetate, the combined organics washed with brine, dried over sodium sulfate, and concentrated in vacuo to give a colorless transparent solid. The residue was recrystallized from diisopropyl ether/hexane to give after filtration, 275 mg (37%) of 15-deoxo-15-hydroxyrapamycin, mp 118 - 122°C.
The spectral data follow: $^1$HNMR ($CDCl_3$, 400 MHz) δ 4.42 (d, 1H, -OH), 4.17 (bs, 1H, anomeric OH), 3.40 (s, 3H, $OCH_3$), 3.39 (s, 3H, $OCH_3$), 3.14 (s, 3H, $OCH_3$), 1.75 (s, 3H, $CH_3C$=C), 1.60 (s, 3H, $CH_3C$=C); $^{13}$C NMR ($CDCl_3$, 100 MHz) δ 216.0, 209.0, 174.5, 169.9; IR (KBr) 3440, 2940, 2860, 1740, 1720, 1630, 1450, 1380, 1195, 1090 $cm^{-1}$; MS (neg. ion FAB) 914 (M-H), 592, 339, 184, 168 (100).

| | | | |
|---|---|---|---|
| Analysis Calcd. for $C_{51}H_{81}NO_{13}$ | C, 66.86; | H, 8.91; | N, 1.53 |
| Found | C, 66.46; | H, 9.03; | N, 1.36 |

Example 2.

15,27-di(deoxo)-15,27-di(hydroxy)rapamycin

To a solution of 1.43 g (1.56 mmol) of rapamycin in 20 mL of dry THF at -78°C was added dropwise 6.87 mL (6.87 mmol) of a 1.0 M solution of DIBAL in toluene. The reaction was slowly warmed to -20° over a 4 h period and then quenched by stirring over 1.0 N HCl for 20 min. The reaction mixture was extracted three times with ethyl acetate, the organic layers combined, washed with brine, dried over sodium sulfate, filtered, and concentrated in vacuo to give a foamy solid. The residue was purified via HPLC chromatography (2 in Dynamax silica column, 100% ethyl acetate, 35 mL/min) gave 90 mg (6%) of 15,27-bis(deoxy)- 15,27-bis(hydroxy)rapamycin.
The spectral data follow: $^1$H NMR ($CDCl_3$, 400 MHz) δ 4.35 (m, 1H, -OH), 4.17 (bs, 1H, anomeric OH), 3.39 (s, 3H, $OCH_3$), 3.33 (s, 3H, $OCH_3$), 3.12 (s, 3H, $OCH_3$), 1.71 (s, 3H, $CH_3C$=C), 1.58 (s, 3H, $CH_3C$=C); IR (KBr) 3435, 2930, 2870, 1740, 1720, 1640, 1450, 1380, 1195, 1090 $cm^{-1}$; MS (neg. ion FAB) 917 (M-).

| | | | |
|---|---|---|---|
| Analysis Calcd. for $C_{51}H_{83}NO_{13}$ • $2H_2O$ | C, 64.19; | H, 9.19 | N, 1.47 |
| Found | C, 64.07; | H, 8.28; | N, 1.62 |

Example 3

15,27-di(deoxo)-15,27-di(hydroxy)rapamycin

In the above procedure, a second fraction, 60 mg (4%) of 15,27-bis(deoxo)-15,27-bis(hydroxy)rapamycin, was also collected.
The spectral data follow: $^1$H NMR ($CDCl_3$, 400 MHz) δ 4.39 (m, 1H, -OH), 4.21 (m, 1H, anomeric OH), 3.41 (s, 3H, $OCH_3$), 3.40 (s, 3H, $OCH_3$), 3.15 (s, 3H, $OCH_3$), 1.73 (s, 3H, $CH_3C$=C), 1.60 (s, 3H, $CH_3C$=C); IR (KBr) 3440, 2930, 2880, 1740, 1720, 1640, 1450, 1380, 1190, 1090 $cm^{-1}$; MS (neg. ion FAB) 917 (M-), 359, 168 (100).

| | | | |
|---|---|---|---|
| Analysis Calcd. for $C_{51}H_{83}NO_{13}$ • $1.5H_2O$ | C, 64.80; | H, 9.17; | N, 1.48 |
| Found | C, 64.69; | H, 8.86; | N, 1.59 |

**Example 4**

33-Deoxy-33-hydroxyrapamycin

A solution of rapamycin (1.05 g, 1. 15 mmol) in tetrahydrofuran (40 mL) was treated with powdered sodium triacetoxyborohydride (0.52 g, 2.46 mmol) and stirred under nitrogen at room temperature for 2h. The reaction mixture was then stored at -20°C for seven days. TLC indicated the reaction to be about 70% complete so the reaction mixture was partitioned between diethyl ether (50 mL) and IN HCl (50 mL). The organic layer was washed with brine (50 mL), dried ($MgSO_4$), concentrated and chromatographed on silica gel ( 3.9 x 15 cm , ethyl acetate). The product fractions which contained unreacted rapamycin were combined and chromatographed by Prep HPLC (8 μm silica gel, 4.1 mm x 30 cm , 1/1: THF/hexane). The clean fractions were combined and dissolved in aqueous methanol. The mixture was concentrated to effect crystallization and the powder was filtered off to give 200 mg of the R-isomer. Spectral Data follows. IR (KBr) 1680, 1730, 2920, 3430 $cm^{-1}$, $^1$H-NMR ($CDCl_3$) δ 1.28 (3H, t, J=7.14 Hz), 1.65 (3H, s), 1.74 (3H, s), 3.14 (3H, s), 3.34 (3H, s), 3.41 (3H, s), 4.20 (2H, q, J=7.14 Hz), 4.30 (2H, dd); Mass Spec (neg. ion FAB) m/z 999 (94%), 590 (15%), 407 (16%), 379 (4%), 253 (6%), 167 (100%).

| | | | |
|---|---|---|---|
| Analysis Calcd. for $C_{55}H_{85}NO_{15}$ • $0.5H_2O$ | C, 65.45; | H, 8.59; | N, 1.39 |
| Found | C, 65.29; | H, 8.64; | N, 1.60 |

**Example 5**

15, 27, 33-tri(deoxo)-15, 27, 33-tri(hydroxy)rapamycin

Following the reduction procedures of Example 4, the title compound is obtained from 15, 27-di(deoxo)-15,27-di(hydroxy)rapamycin as prepared in Examples 2 and 3.

**Example 6**

15,33-di(deoxo)- 15,33-di(hydroxy)rapamycin

Following the reduction procedures of Example 4, the title compound is obtained from 15-deoxo-15-hydroxyrapamycin as prepared in Example 1.

Immunosuppressive activity was evaluated in an in vitro standard pharmacological test procedure to measure lymphocyte proliferation (LAF) and in two in vivo standard pharmacological test procedures. The first in vivo procedure was a popliteal lymph node (PLN) test procedure which measured the effect of compounds of this invention on a mixed lymphocyte reaction and the second in vivo procedure evaluated the survival time of a pinch skin graft.

The comitogen-induced thymocyte proliferation procedure (LAF) was used as an in vitro measure of the immunosuppressive effects of representative compounds. Briefly, cells from the thymus of normal BALB/c mice are cultured for 72 hours with PHA and IL-1 and pulsed with tritiated thymidine during the last six hours. Cells are cultured with and without various concentrations of rapamycin, cyclosporin A, or test compound. Cells are harvested and incorporated; radioactivity is determined. Inhibition of lymphoproliferation is assessed in percent change in counts per minute from non-drug treated controls. The results are expressed by the following ratio, or as the percent inhibition of lymphoproliferation of 1 μM.

$$\frac{^3\text{H-control thymus cells - H}^3\text{rapamycin-treated thymus cells}}{^3\text{H-control thymus cells - H}^3\text{-test compound-treated cells}}$$

A mixed lymphocyte reaction (MLR) occurs when lymphoid cells from genetically distinct animals are combined in tissue culture. Each stimulates the other to undergo blast transformation which results in increased DNA synthesis that can be quantified by the incorporation of tritiated thymidine. Since stimulating a MLR is a function of disparity at Major Histocompatibility antigens, an in vivo popliteal lymph node (PLN) test procedure closely correlates to host vs. graft disease. Briefly, irradiated spleen cells from BALB/c donors are injected into the right hind foot pad of recipient C3H mice. The drug is given daily, p.o. from Day 0 to Day 4. On Day 3 and Day 4, tritiated thymidine is given i.p., b.i.d. On Day 5, the hind popliteal lymph nodes are removed and dissolved, and radioactivity counted. The corresponding left

PLN serves as the control for the PLN from the injected hind foot. Percent suppression is calculated using the non-drug treated animals as allogenic control. Rapamycin at a dose of 6 mg/kg, p.o. gave 86% suppression, whereas cyclosporin A at the same dose gave 43% suppression. Results are expressed by the following ratio:

$$\frac{{}^{3}\text{H-PLN cells control C3H mouse - }{}^{3}\text{H-PLN cells rapamycin-treated C3H mouse}}{{}^{3}\text{H-PLN cells control C3H mouse - }{}^{3}\text{H-PLN cells test compound-treated C3H mouse}}$$

The second in vivo test procedure is designed to determine the survival time of pinch skin graft from male DBA/2 donors transplanted to male BALB/c recipients. The method is adapted from Billingham R.E. and Medawar P.B., J. Exp. Biol. 28:385-402, (1951). Briefly, a pinch skin graft from the donor is grafted on the dorsum of the recipient as a homograft, and an autograft is used as control in the same region. The recipients are treated with either varying concentrations of cyclosporin A as test control or the test compound, intraperitoneally. Untreated recipients serve as rejection control. The graft is monitored daily and observations are recorded until the graft becomes dry and forms a blackened scab. This is considered as the rejection day. The mean graft survival time (number of days ± S.D.) of the drug treatment group is compared with the control group.

The following table summarizes the results of the compounds of this invention in these three standard test procedures.

Table 1

| Compound | LAF[1] | PLN[2] | Skin Graft[3] |
|---|---|---|---|
| Example 1 | 0.58 | -2.47 po, -0.07 ip | 9.8 ± 1.0 |
| Example 2 | 0.67 | - | 9.2 ± 0.41 |
| Example 3 | 0.84 | 0.92 ip | 9.5 ± 5.5 |
| Example 4 | 4.7 | 0.59 | 10.0 |
| Rapamycin | 3.3 | 1 | 12.5 |

[1] $IC_{50}$ (nM)
[2] Activity relative to Rapamycin
[3] Mean survival days

The results of these standard pharmacological test procedures demonstrate immunosuppressive activity both in vitro and in vivo for the compounds of this invention. Positive ratios in the LAF and PLN test procedures indicate suppression of T cell proliferation. As transplanted pinch skin grafts are typically rejected with 6-7 days without the use of an immunosuppressive agent, the increased survival time of the skin graft when treated with the compounds of this invention further demonstrates their utility as immunosuppressive agents.

Antifungal activity of the compounds of this invention was measured against 5 strains of Candida albicans using a plate test procedure for measurement of inhibition. The following represents the typical procedure used. The compound to be tested was placed on sterile dried 1/4" plate disks, and allowed to dry. Agar plates were seeded with fungi and allowed to solidify. The impregnated disks were placed on the seeded Agar surface and incubated for the time required for the particular culture. Results are expressed in MIC (µg/ml) to inhibit growth. The results of this test procedure showed that the compounds of this invention have antifungal activity.

The compounds of Examples 1 and 4 had the following minimal inhibitory concentrations (50%) against 5 strains of Candida albicans.

Table II

| Anti-Candida Activity (µg/mL)* | | | | | |
|---|---|---|---|---|---|
| Compound | ATCC 10231 | ATCC 38246 | ATCC 38247 | ATCC 38248 | 3669 |
| Example 1 | 0.025 | 0.2 | 0.025 | 0.05 | 0.2 |
| Rapamycin | 0.003 | 0.025 | 0.003 | 0.006 | 0.025 |
| Example 4 | 0.05 | >0.4 | 0.03 | 0.006 | 0.25 |
| Rapamycin | 0.03 | 0.75 | 0.1 | 0.2 | 0.4 |

*Minimal Inhibitory Concentration (50%), Rapamycin run as standard against test compound.

Based on the results of these standard pharmacological test procedures, the compounds are useful in the treatment of transplantation rejection such as, heart, kidney, liver, bone marrow, and skin transplants; autoimmune diseases such as, lupus, rheumatoid arthritis, diabetes mellitus, myasthenia gravis, and multiple sclerosis; and diseases of inflammation such as, psoriasis, dermatitis, eczema, seborrhea, inflammatory bowel disease; and fungal infections.

The compounds may be administered neat or with a pharmaceutical carrier to a mammal in need thereof. The pharmaceutical carrier may be solid or liquid and the active compound shall be a therapeutically effective amount.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellent.

Liquid pharmaceutical compositions which are sterile solutions or suspensions can be utilized by, for example, intramuscular, intraperitoneal or subcutaneous injection. Sterile solutions can also be administered intravenously. The compound can also be administered orally either in liquid or solid composition form.

Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form. The dosage to be used in the treatment must be subjectively determined by the attending physician.

In addition, the compounds of this invention may be employed as a solution, cream, or lotion by formulation with pharmaceutically acceptable vehicles containing 0.1-0.5 percent, preferably 2%, of active compound which may be administered to a fungally affected area.

Accordingly this invention also provides a pharmaceutical composition comprising a compound of formula I or a pharmaceutically acceptable salt thereof and a pharmaceutically acceptable carrier.

## Claims

**Claims for the following Contracting States : AT, BE, CH, DE, DK, FR, GB, IT, LI, LU, NL, PT, SE**

1. A compound having the formula:

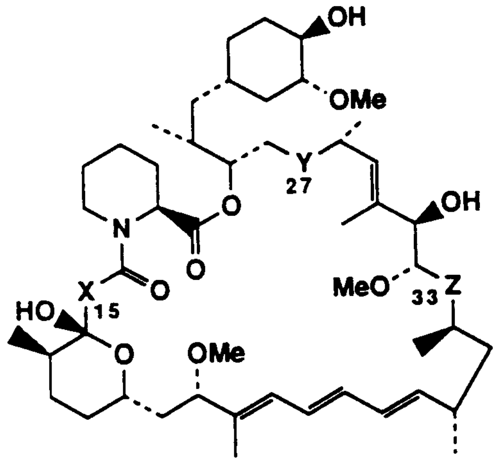

wherein X, Y, and Z are -CO or -CHOH- and where at least one of X or Z must be -CHOH- providing that if Y is CHOH then X is CHOH or a pharmaceutically acceptable salt thereof.

2. A compound according to claim 1 which is 15-Deoxo-15-hydroxyrapamycin or a pharmaceutically acceptable salt thereof.

3. A compound according to claim 1 which is 15,27-Bis(deoxo)-15,27-bis(hydroxy)rapamycin or a pharmaceutically acceptable salt thereof.

4. A compound according to claim 1 which is 33-Deoxy-33-hydroxyrapamycin or a pharmaceutically acceptable salt thereof.

5. A compound according to claim 1 which is 33-Deoxy-33-(R)-hydroxyrapamycin or a pharmaceutically acceptable salt thereof.

6. A compound according to claim 1 which is 15,33-Bis(deoxy)-15,33-bis(hydroxy)rapamycin or a pharmaceutically acceptable salt thereof.

7. A compound according to claim 1 which is 15,27,33-Tri(deoxy)-15,27,33-tri(hydroxy)rapamycin or a pharmaceutically acceptable salt thereof.

8. A process for preparing a compound having the formula I as defined in Claim 1 wherein X, Y and Z are -CO- or -CHOH- and where at least one of X or Z must be -CHOH- or a pharmaceutically acceptable salt thereof, which comprises reducing rapamycin or a 31,42- OH protected derivative thereof using a reducing agent, or two reducing agents sequentially in either order, selected from diisobutylaluminium hydride or similar standard ketone reducing agent and a β-hydroxyketone reducing agent and removing any protecting groups present to give a compound of formula I, if desired isolating as a pharmaceutically acceptable salt thereof.

9. A pharmaceutical composition comprising a pharmaceutical carrier and a compound of formula I as defined in Claim 1.

10. A compound of formula I as claimed in any one of Claims 1 to 7 for use as pharmaceutical.

11. Use of a compound of formula I as claimed in Claim 1 in the manufacture of a medicament for use as an immunosuppressant, antiinflammatory or antifungal agent.

**Claims for the following Contracting States: ES, GR**

1. A process for preparing compound having the formula:

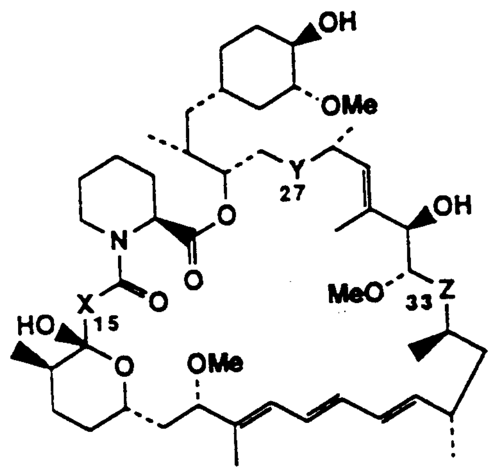

(I)

wherein X, Y, and Z are -CO or -CHOH- and where at least one of X or Z must be -CHOH- providing that if Y is CHOH then X is CHOH or a pharmaceutically acceptable salt thereof, which comprises reducing rapamycin or a 31,42- OH protected derivative thereof using a reducing agent, or two reducing agents sequentially in either order, selected from (a) diisobutylaluminium hydride or similar standard ketone reducing agent and (b) a β-hydroxyketone reducing agent, and removing any protecting groups present to give a compound of formula I, if desired isolating as a pharmaceutically acceptable salt thereof.

2. A process as claimed in Claim 1 in which the β-hydroxyketone reducing agent is sodium triacetoxyborohydride.

3. A process as claimed in Claim 1 in which the product is 15-deoxo-15-hydroxyrapamycin or a pharmaceutically acceptable salt thereof.

4. A process as claimed in Claim 1 in which the product is 15,27-bis(deoxo)-15,27-bis(hydroxy)rapamycin or a pharmaceutically acceptable salt thereof.

5. A process as claimed in Claim 1 in which the product is 33-deoxy-33-hydroxyrapamycin or a pharmaceutically acceptable salt thereof.

6. A process as claimed in Claim 1 in which the product is 33-deoxy-33-(R)-hydroxyrapamycin or a pharmaceutically acceptable salt thereof.

7. A process as claimed in Claim 1 in which the product is 15,33-bis(deoxy)-15,33-bis(hydroxy)rapamycin or a pharmaceutically acceptable salt thereof.

8. A process as claimed in Claim 1 in which the product is 15,27,33-tri(deoxy)-15,27,33-tri(hydroxy)rapamycin or a pharmaceutically acceptable salt thereof.

9. A process for preparing a pharmaceutical composition which comprises bringing a pharmaceutical carrier and a compound of formula I as defined in Claim 1 into association.

10. Use of a compound of formula I as defined in Claim 1 in the manufacture of a medicament for use as an immunosuppressant, antiinflammatory or antifungal agent.

**Patentansprüche**

**Patentansprüche für folgende Vertragsstaaten : AT, BE, CH, DE, DK, FR, GB, IT, LI, LU, NL, PT, SE**

1. Verbindung der Formel:

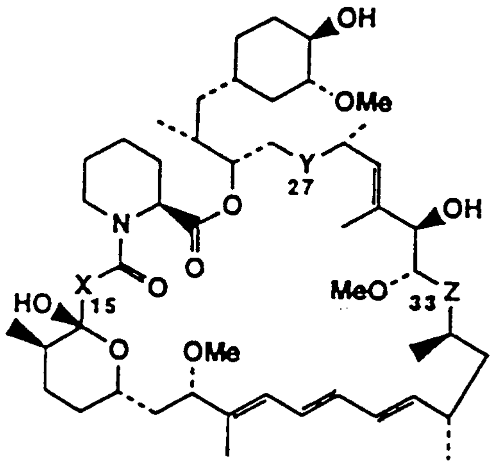

worin X, Y und Z für -CO oder -CHOH- stehen und wobei mindestens eines von X oder Z für -CHOH- stehen muß, vorausgesetzt daß falls Y für CHOH steht, X für CHOH steht, oder ein pharmazeutisch annehmbares Salz davon.

2. Verbindung gemäß Anspruch 1, welche 15-Desoxo-15-hydroxyrapamycin oder ein pharmazeutisch annehmbares Salz davon ist.

3. Verbindung gemäß Anspruch 1, welche 15,27-Bis(desoxo)-15,27-bis(hydroxy)rapamycin oder ein pharmazeutisch annehmbares Salz davon ist.

4. Verbindung gemäß Anspruch 1, welche 33-Desoxy-33-hydroxyrapamycin oder ein pharmazeutisch annehmbares Salz davon ist.

5. Verbindung gemäß Anspruch 1, welche 33-Desoxy-33-(R)-hydroxyrapamycin oder ein pharmazeutisch annehmbares Salz davon ist.

6. Verbindung gemäß Anspruch 1, welche 15,33-Bis(desoxy)-15,33-bis(hydroxy)rapamycin oder ein pharmazeutisch annehmbares Salz davon ist.

7. Verbindung gemäß Anspruch 1, welche 15,27,33-Tri(desoxy)-15,27,33-tri(hydroxy)rapamycin oder ein pharmazeutisch annehmbares Salz davon ist.

8. Verfahren zum Herstellen einer Verbindung mit der Formel I, wie in Anspruch 1 definiert, worin X, Y und Z für -CO- oder -CHOH- stehen und wo mindestens eines von X oder Z für -CHOH- stehen muß, oder ein pharmazeutisch annehmbares Salz davon, welches umfaßt: Reduzieren von Rapamycin oder einem 31,42-OH geschützten Derivat davon unter Verwendung eines Reduktionsmittels oder zweier Reduktionsmittel, aufeinanderfolgend in jeder Reihenfolge, ausgewählt aus Diisobutylaluminiumhydrid oder ähnlichem Standard-Keton-Reduktionsmittel und einem β-Hydroxyketon-Reduktionsmittel, und Entfernen jeder anwesenden Schutzgruppen, um eine Verbindung der Formel I zu ergeben, falls gewünscht Isolieren als ein pharmazeutisch annehmbares Salz davon.

9. Pharmazeutische Zusammensetzung, umfassend einen pharmazeutischen Träger und eine Verbindung der Formel I, wie in Anspruch I definiert.

10. Verbindung der Formel I, wie in einem der Ansprüche 1 bis 7 beansprucht, zur Verwendung als Pharmazeutikum.

11. Verwendung einer Verbindung der Formel I, wie in Anspruch I beansprucht, bei der Herstellung eines Medikaments zur Verwendung als ein Immunsuppressiv-, Antientzündungs- oder Antipilzmittel.

**Patentansprüche für folgende Vertragsstaaten : ES, GR**

1. Verfahren zum Herstellen von Verbindung der Formel

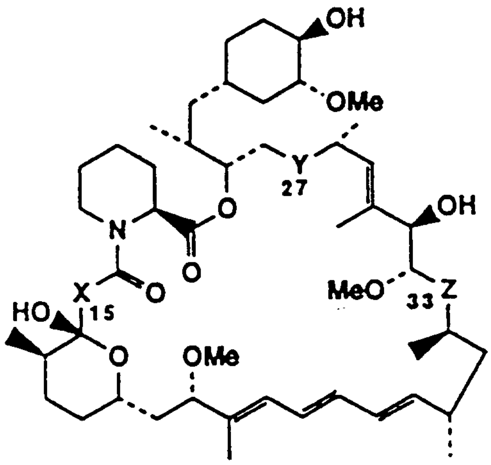

(I),

worin X, Y und Z für -CO oder -CHOH- stehen und wo mindestens eines von X oder Z für -CHOH- stehen muß, vorausgesetzt daß falls Y für CHOH steht, X für CHOH steht, oder ein pharmazeutisch annehmbares Salz davon, welches umfaßt: Reduzieren von Rapamycin oder einem 31,42-OH geschützten Derivat davon unter Verwendung eines Reduktionsmittels oder zweier Reduktionsmittel, aufeinanderfolgend in jeder Reihenfolge, ausgewählt aus (a) Diisobutylaluminiumhydrid oder ähnlichem Standard-Keton-Reduktionsmittel und (b) einem β-Hydroxyketon-Reduktionsmittel, und Entfernen jeder anwesenden Schutzgruppe, um eine Verbindung der Formel I zu ergeben, falls gewünscht Isolieren als ein pharmazeutisch annehmbares Salz davon.

2. Verfahren wie in Anspruch 1 beansprucht, bei welchem das β-Hydroxyketon-Reduktionsmittel Natriumtriacetoxyborhydrid ist.

3. Verfahren wie in Anspruch 1 beansprucht, bei welchem das Produkt 15-Desoxo-15-hydroxyrapamycin oder ein pharmazeutisch annehmbares Salz davon ist.

4. Verfahren wie in Anspruch 1 beansprucht, bei welchem das Produkt 15,27-Bis(desoxo)-15,27-bis(hydroxy)rapamycin oder ein pharmazeutisch annehmbares Salz davon ist.

5. Verfahren wie in Anspruch 1 beansprucht, bei welchem das Produkt 33-Desoxy-33-hydroxyrapamycin oder ein pharmazeutisch annehmbares Salz davon ist.

6. Verfahren wie in Anspruch 1 beansprucht, bei welchem das Produkt 33-Desoxy-33-(R)-hydroxyrapamycin oder ein pharmazeutisch annehmbares Salz davon ist.

7. Verfahren wie in Anspruch 1 beansprucht, bei welchem das Produkt 15,33-Bis(desoxy)-15,33-bis(hydroxy)rapamycin oder ein pharmazeutisch annehmbares Salz davon ist.

8. Verfahren wie in Anspruch 1 beansprucht, bei welchem das Produkt 15,27,33-Tri(desoxy)-15,27,33-tri(hydroxy)rapamycin oder ein pharmazeutisch annehmbares Salz davon ist.

9. Verfahren zum Herstellen einer pharmazeutischen Zusammensetzung, welches umfaßt: in Verbindung bringen eines pharmazeutischen Trägers und einer Verbindung von Formel I, wie in Anspruch I definiert.

10. Verwendung einer Verbindung der Formel I, wie in Anspruch I definiert, bei der Herstellung eines Medikaments zur

Verwendung als ein Immunsuppressiv-, Antientzündungs- oder Antipilzmittel.

## Revendications

**Revendications pour les Etats contractants suivants : AT, BE, CH, DE, DK, FR, GB, IT, LI, LU, NL, PT, SE**

1. Composé ayant la formule :

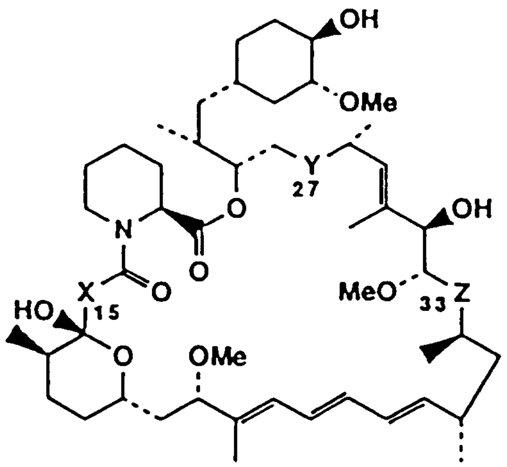

dans laquelle X, Y et Z sont -CC ou -CHOH- et où au moins un des X ou Z doit être -CHOH- à condition que si Y est CHOH alors X est CHOH ou un sel pharmaceutiquement acceptable de celui-ci.

2. Composé selon la revendication 1 qui est la 15-déoxo-15-hydroxyrapamycine ou un sel pharmaceutiquement acceptable de celle-ci.

3. Composé selon la revendication 1 qui est la 15,27-bis(déoxo)-15,27-bis(hydroxy)rapamycine ou un sel pharmaceutiquement acceptable de celle-ci.

4. Composé selon la revendication 1 qui est la 33-déoxy-33-hydroxyrapamycine ou un sel pharmaceutiquement acceptable de celle-ci.

5. Composé selon la revendication 1 qui est la 33-déoxy-33-(R)-hydroxyrapamycine ou un sel pharmaceutiquement acceptable de celle-ci.

6. Composé selon la revendication 1 qui est la 15,33-bis(déoxy)-15,33-bis(hydroxy)rapamycine ou un sel pharmaceutiquement acceptable de celle-ci.

7. Composé selon la revendication 1 qui est la 15,27,33-tri(déoxy)-15,27,33-tri(hydroxy)rapamycine ou un sel pharmaceutiquement acceptable de celle-ci.

8. Procédé de préparation d'un composé ayant la formule I comme défini dans la revendication 1 dans lequel X, Y et Z sont -CO- ou -CHOH- et où au moins un des X ou Z doit être -CHOH- ou un sel pharmaceutiquement acceptable de celui-ci, qui comprend la réduction de la rapamycine ou d'un dérivé protégé 31,42-OH de celle-ci en utilisant un agent réducteur, ou deux agents réducteurs séquentiellement dans un ordre quelconque, choisis parmi l'hydrure de diisobutylaluminium ou un agent réducteur similaire de cétone standard et un agent réducteur de β-hydroxycétone et l'élimination de tout groupement protecteur présent pour donner un composé de formule I, si souhaité en l'isolant sous forme d'un sel pharmaceutiquement acceptable de celui-ci.

9. Composition pharmaceutique comprenant un support pharmaceutique et un composé de formule I comme défini dans la revendication 1.

10. Composé de formule I selon l'une quelconque des revendications 1 à 7 pour l'utilisation comme produit pharmaceutique.

**11.** Utilisation d'un composé de formule I selon la revendication 1 dans la fabrication d'un médicament pour l'utilisation comme agent immunosuppresseur, anti-inflammatoire ou antifongique.

**Revendications pour les Etats contractants suivants : ES, GR**

**1.** Procédé de préparation d'un composé ayant la formule

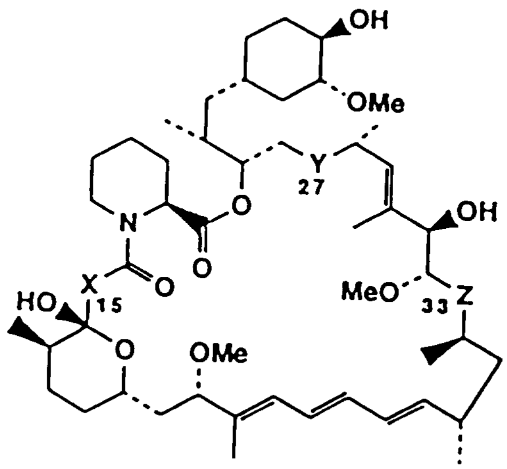

dans laquelle X, Y et Z sont -CO ou -CHOH- et où au moins un des X ou Z doit être -CHOH- à condition que si Y est CHOH alors X est CHOH ou un sel pharmaceutiquement acceptable de celui-ci, qui comprend la réduction de la rapamycine ou d'un dérivé protégé 31,42-OH de celle-ci en utilisant un agent réducteur, ou deux agents réducteurs séquentiellement dans un ordre quelconque, choisis parmi (a) l'hydrure de diisobutylaluminium ou un agent réducteur similaire de cétone standard et (b) un agent réducteur de β-hydroxycétone et l'élimination de tout groupement protecteur présent pour donner un composé de formule I, si souhaité en l'isolant sous forme de sel pharmaceutiquement acceptable de celui-ci.

**2.** Procédé selon la revendication 1 dans lequel l'agent réducteur de β-hydroxycétone est le triacétoxyborohydrure de sodium.

**3.** Procédé selon la revendication 1 dans lequel le produit est la 15-déoxo-15-hydroxyrapamycine ou un sel pharmaceutiquement acceptable de celle-ci.

**4.** Procédé selon la revendication 1 dans lequel le produit est la 15,27-bis(déoxo)-15,27-bis(hydroxy)rapamycine ou un sel pharmaceutiquement acceptable de celle-ci.

**5.** Procédé selon la revendication 1 dans lequel le produit est la 33-déoxy-33-hydroxyrapamycine ou un sel pharmaceutiquement acceptable de celle-ci.

**6.** Procédé selon la revendication 1 dans lequel le produit est la 33-déoxy-33-(R)-hydroxyrapamycine ou un sel pharmaceutiquement acceptable de celle-ci.

**7.** Procédé selon la revendication 1 dans lequel le produit est la 15,33-bis(déoxy)-15,33-bis(hydroxy)rapamycine ou un sel pharmaceutiquement acceptable de celle-ci.

**8.** Procédé selon la revendication 1 dans lequel le produit est la 15,27,33-tri(déoxy)-15,27,33-tri(hydroxy)rapamycine ou un sel pharmaceutiquement acceptable de celle-ci.

**9.** Procédé de préparation d'une composition pharmaceutique qui comprend la mise en association d'un support pharmaceutique et d'un composé de formule I comme défini dans la revendication 1.

**10.** Utilisation d'un composé de formule I comme défini dans la revendication 1 dans la fabrication d'un médicament pour l'utilisation comme agent immunosuppresseur, anti-inflammatoire ou antifongique.